Europäisches Patentamt

European Patent Office

Office européen des brevets

(19)

(11) Numéro de publication: **0 148 686**
**A2**

# (12) DEMANDE DE BREVET EUROPEEN

(21) Numéro de dépôt: 84402627.8

(22) Date de dépôt: 18.12.84

(51) Int. Cl.⁴: **C 12 Q 1/68, C 12 N 15/00**

(30) Priorité: 21.12.83 FR 8320487

(43) Date de publication de la demande: 17.07.85
Bulletin 85/29

(84) Etats contractants désignés: **AT BE CH DE FR GB IT LI LU NL SE**

(71) Demandeur: **CENTRE NATIONAL DE LA RECHERCHE SCIENTIFIQUE (C.N.R.S.), 15, Quai Anatole France, F-75007 Paris (FR)**
Demandeur: **INSTITUT PASTEUR, 28, rue du Docteur Roux, F-75715 Paris Cedex 15 (FR)**

(72) Inventeur: **Ricquier, Daniel, 75, rue de Villiers, F-78300 Poissy (FR)**
Inventeur: **Bouillaud, Frédéric, 54, Allée dela Forêt, F-92360 Meudon-la-Foret (FR)**
Inventeur: **Thibault, Jean, 14, rue Mouton-Duvernet, F-75014 Paris (FR)**
Inventeur: **Weissenbach, Jean, 163, rue de Vaugirard, F-75015 Paris (FR)**

(74) Mandataire: **Warcoin, Jacques et al, Cabinet Régimbeau 26, avenue Kléber, F-75116 Paris (FR)**

(54) Sonde d'ADNc reconnaissant l'ARNm de la protéine découplante des mitochondries du tissu adipeux brun des mammifères, procédé de préparation et application.

(57) La présente invention concerne une sonde constituée en partie d'une séquence d'acides nucléiques, cette séquence d'acide nucléique reconnaissant spécifiquement l'ARNm et/ou le gène de la protéine découplante des mitochondries du tissu adipeux brun des mammifères.

EP 0 148 686 A2

0148686

Sonde d'ADNc reconnaissant l'ARNm de la protéine
découplante des mitochondries du tissu adipeux brun
des mammifères, procédé de préparation et application.

La présente invention concerne plus particulièrement
la mise en évidence et le dosage des gènes et des mARN
codant pour la synthèse de la protéine découplante.

Chez les mammifères, animaux homéothermes, le
métabolisme de base n'est pas suffisant pour assurer la
régulation thermique dans des situations où le besoin de
thermogénèse existe. Ce besoin de production de chaleur
se rencontre chez les individus exposés au froid, chez les
nouveau-nés et chez les animaux hibernants lors du réveil.
Pour produire de la chaleur les organismes disposent de
deux systèmes : le frisson thermique et la thermogénèse
sans frisson (ou thermogénèse chimique). Il a été constaté
que la capacité de thermogénèse sans frisson chez beaucoup
d'espèces est liée à la présence d'un tissu particulier,
le tissu adipeux brun (TAB), appelé aussi graisse brune.

Le TAB est localisé essentiellement dans les
régions interscapulaire, péricervicale, axillaire,
thoracique et périrénale. Cytologiquement l'adipocyte brun
diffère de l'adipocyte blanc par ses nombreuses gouttelettes
lipidiques (graisse multiloculaire) et par un chondriome
très abondant (les mitochondries comportant de très
nombreuses crêtes). Le tissu est abondamment vascularisé
et innervé par des fibres orthosympathiques. Lorsque la
stimulation de la thermogénèse est ordonnée par le cerveau
et l'hypothalamus, l'innervation orthosympathique du TAB active le tissu, la lipolyse est stimulée et les acides gras libres sont oxydés par les mitochondries, de la chaleur est produite et dissipée dans l'organisme par la circulation sanguine.

Le TAB a une très forte capacité oxydative vis à vis des graisses, c'est pourquoi son rôle a été évoqué récemment dans la régulation du poids corporel ; cette hypothèse est fondée sur des observations faites chez des animaux génétiquement ou expérimentalement obèses et chez des animaux suralimentés.

Dans des mitochondries "normales", il existe un couplage étroit entre l'oxydation d'un substrat par la chaine respiratoire, réaction qui libère de l'énergie, et la phosphorylation de l'ADP en ATP, réaction qui consomme de l'énergie. C'est d'ailleurs la non-disponibilité de l'ADP pour cette dernière réaction qui limite la vitesse de respiration des mitochondries : les mitochondries sont dites couplées, elles ne dégagent alors que peu de chaleur. Dans le cas exceptionnel des mitochondries du TAB, le couplage obligatoire oxydation/phosphorylation n'existe plus, elles sont découplées et alors capables d'oxyder rapidement des substrats sans être obligées de phosphoryler l'ADP ; l'énergie libérée par l'oxydation rapide des acides gras libres est alors dissipée sous forme de chaleur.

Lorsque des rats sont adaptés au froid, le TAB s'hypertrophie, le chondriome se développe et les mitochondries sont très découplées. Il avait été observé au cours de cette adaptation, qu'au niveau de la membrane interne mitochondriale la proportion d'un polypeptide de poids moléculaire apparent 32 000 s'élève très significativement, ce polypeptide pouvant alors représenter 10 % des protéines mitochondriales (Ricquier & Kader 1976).

Peu de temps après, cherchant à identifier le site de fixation de nucléotides recouplants, Heaton et al. (1978) observèrent que ceux-ci se liaient spécifiquement à un polypeptide membranaire de poids moléculaire apparent 32 000.

Des arguments variés, électrophorétiques et immunologiques ont confirmé la spécificité tissulaire de cette protéine et sa non-identité à d'autres composants connus de la membrane interne mitochondriale (Ricquier et al. 1979, 1982 a, 1983 a ; Cannon et al. 1982) ; la purification de la protéine a été mise au point (Lin et al. 1980, Ricquier et al. 1982 a). Il est maintenant admis que le découplage naturel des mitochondries du TAB et leur fonction thermogénique sont liés à la présence de cette protéine découplante. Certains arguments indiquent que la protéine 32 000 pourrait former des canaux ioniques (sensibles aux nucléotides) dans la membrane interne. Par ailleurs, il a été récemment montré, soit sur des rats adaptés au froid et sympathectomisés (Mory et al. 1982), soit sur des rats porteurs de phéochromocytomes (Ricquier et al. 1983 b) que des facteurs orthosympathiques contrôlent le taux de la protéine découplante.

La réalisation d'une sonde spécifique permettant la mise en évidence du gène et du mARN codant pour cette protéine découplante des mitochondries est utile dans de nombreux domaines.

Des travaux récents démontrent que le niveau de thermogénèse dans le TAB peut réguler le poids corporel d'animaux obèses. Le TAB peut brûler les graisses de l'organisme et lutter contre la prise de poids ; chez les animaux obèses, le TAB n'est pas fonctionnel. Ainsi, actuellement, plusieurs équipes dont celles de plusieurs laboratoires pharmaceutiques étrangers, cherchent à fabriquer une drogue qui pourrait spécifiquement induire l'apparation du TAB, induire la synthèse de la protéine découplante et donc induire une thermogénèse qui pourrait

0148686

s'opposer au gain de poids. On ne sait si chez l'adulte humain le TAB peut jouer un rôle, mais il est connu que l'obèse humain a des capacités thermogénétiques diminuées. L'utilisation de la sonde ADNc selon l'invention est utile dans la recherche d'un nouveau médicament thermogénétique et efficace contre l'obésité.

Beaucoup de cellules cancéreuses font de la chaleur dont l'origine est mal connue. Il n'est pas interdit de penser que dans certains cas de cancérogenèse, la synthèse de la protéine découplante thermogénique est induite. Une telle étude ne peut se faire qu'en utilisant les sondes d'ADNc décrites ci-après.

- En règle générale, ce type de sonde pourrait être utilisé pour essayer de comprendre et de traiter les situations pathologiques où il y a déséquilibre énergétique (obésité, cachexie, hyperthermie, hypothermie ...).

- Enfin, ce genre de sonde d'ADNc pourrait être utile à la mise au point de systèmes bactériens capables de constituer un chauffage biologique.

C'est pourquoi la présente invention concerne :

une sonde constituée en partie d'une séquence d'acides nucléiques, cette séquence d'acides nucléiques reconnaissant spécifiquement le gène et/ou les mARN de la protéine découplante des mitochondries du TAB (ci-après protéine découplante). De préférence la séquence d'acides nucléiques est une séquence d'ADNc.

Cette sonde peut comporter en outre tout ou partie de l'ADN d'un plasmide bactérien tel que pBR 327 et elle peut en outre comporter un marqueur radioactif qui permettra sa détection et son dosage éventuel.

L'ADN du plasmide bactérien (comportant de préférence une origine de réplication et un gène de résistance à un antibiotique) permet d'assurer la réplication et le maintient de la séquence d'ADNc de la protéine découplante dans des bactéries transformées en particulier E. coli.

C'est pourquoi l'invention concerne également des bactéries incorporant une sonde selon la présente invention.

Enfin la présente invention concerne un procédé d'identification et/ou de dosage des ARNm de la protéine découplante caractérisé en ce qu'on met en contact le milieu comportant lesdits ARNm avec une sonde selon l'invention et que l'on identifie et/ou dose la sonde fixée sur les ARNm. Les dosages quantitatifs peuvent être facilités lorsque la sonde est marquée en particulier lorsqu 'elle est radioactive.

Enfin la présente invention concerne un ensemble de dosage et ou d'identification pour la mise en oeuvre du procédé précédent comportant en particulier une sonde selon l'invention.

D'autres caractéristiques et avantages de la présente invention apparaîtront à la lecture de l'exemple détaillé ci-après.

## I. Obtention de la fraction 17S des ARN poly A de TAB de rat adapté au froid

### 1. Obtention d'ARN totaux

Des rats Spague-Dawley mâles d'environ 200 g sont exposés au froid à 5°C pendant 7 jours.

Le protocole de préparation des ARN est dérivé de celui de Lomedico & Saunders 1976.

Après prélèvement du TAB interscapulaire de ces animaux celui-ci est congelé dans l'azote liquide et conservé à - 80°C ; 5 à 25 g de TAB, représentant quelques dizaines de rats, sont utilisés pour préparer les ARN totaux destinés à la chromatographie sur oligodT-cellulose.

- Broyage et homogénéisation : les TAB congelés sont réduits en poudre par broyage à sec avec un homogénéiseur "Waring Blendor" dont le vase a été refroidi à - 80°C. A cette poudre on ajoute un mélange 50/50 de "tampon Lomedico" et de phénol.

Tampon Lomedico :

|  |  |
|---|---|
| LiCl | : 0,1 M |
| SDS | : 1 % |
| TRIS pH 8.8 | : 0,2 M |
| EDTA | : 25 mM |

L'ensemble est alors homogénéisé dans le même appareil.

- Extraction : l'homogénat est centrifugé (10 000 g pendant 10 minutes à 4°C), on récupère la phase aqueuse ; le mélange phase phénolique et protéines coagulées est retraité par un volume de "tampon Lomedico". Après centrifugation les deux phases aqueuses ainsi obtenues sont réunies. On ajoute alors un égal volume d'un mélange 50/50 (phénol-chloroforme), et par une agitation magnétique nous maintenons à l'état d'émulsion pendant environ une heure, ceci, comme les étapes précédentes, à basse température (≃ 0°C). Cette émulsion est centrifugée (10 000 g, 10 min, 4°C) et la phase aqueuse est récupérée. Ce traitement, au phénol-chloroforme, est répété jusqu'à ce que l'interface soit exempte de protéines coagulées.

- Précipitation : la solution aqueuse ainsi préparée est amenée à 0,3 M NaCl en utilisant du NaCl 5 M, on y ajoute deux volumes d'éthanol absolu préalablement refroidi, l'ensemble est laissé à - 20° C au moins une nuit, ainsi précipités les acides nucléiques se conservent très bien.

- Dissolution : les ARN précipités sont centrifugés (10 000 g ou plus, 20 min, 4°C) le culot d'ARN est lavé une ou deux fois avec une solution (1/3 NaCl 0,3 M, 2/3 éthanol absolu) refroidie à - 20°C, puis dissous dans de l'eau bidistillée stérile à des concentrations de 2 à 5 mg/ml estimées par lecture de la Densité Optique (D.O.) à 260 nm et 280 nm.

2. Chromatographie d'affinité sur oligodT-cellulose

Le montage est nettoyé par circulation d'eau oxygénée puis de NaOH 1 M. Il est ainsi stérilisé et débarrassé de toutes molécules de ribonucléase ou d'ARN qui pourraient s'y trouver.

L'oligodT-cellulose (1 à 2 g pour 1000 UDO d'ARN totaux) est suspendue dans de l'eau distillée stérile puis amenée à 0,1 M NaOH, un jaunissement de l'oligodT-cellulose est normal. On coule alors la colonne qui est rincée à l'eau distillée stérile jusqu'à ce que le pH de sortie avoisine 7. Elle est ensuite rincée avec de "Binding Buffer" (TRIS HCl, pH 7,5 .10 mM, KCl 0,5 M).

Les ARN totaux en solution dans 0,5 M NaCl, 0,1 % SDS, sont déposés sur la colonne, l'absorption à 254 nm est enregistrée avec l'appareil ISCO. Le "Binding Buffer" pousse un grand pic exclu d'ARN non polyA, l'élution à l'eau distillée stérile décroche les ARN polyA et fournit un deuxième pic. Les deux fractions (polyA et non polyA) sont récupérées et précipitées comme décrit précédemment. Le protocole utilisé est celui d'Aviv et Leder (1972).

3. Fractionnement des ARN polyA sur gradients de saccharose

La centrifugation d'un échantillon d'ARN polyA dénaturés déposé au sommet d'un gradient préformé de saccharose permet de séparer les ARN selon leur coefficient de sédimentation (exprimé en unités Svedberg : S). L'étalonnage est obtenu grâce à des standards internes : les ARN ribosomiques 18 S et 28 S encore bien représentés dans les préparations d'ARN polyA. Il existe une relation (non linéaire) qui permet d'estimer le nombre de nucléotides d'un ARN à partir de son coefficient de sédimentation.

Les ARN polyA ont été fractionnés sur deux types de gradients de saccharose.

MILIEU METHYL MERCURE HYDROXYDE (Schweinfest et al. 1982)

a) Traitement de l'échantillon

200 µg d'ARN polyA sont laissés quelques minutes à température ambiante dans un milieu 20 mM méthyl mercure hydroxyde - 10 mM TRIS HCl pH 7,4 - 1 mM EDTA ; puis sont refroidis dans la glace.

b) Centrifugation

L'échantillon traité comme indiqué en (a) est déposé sur un gradient linéaire 10-30 % saccharose dans un milieu 10 mM méthyl mercure hydroxyde - 10 mM TRIS HCl pH 7,4 - 1 mM EDTA. La centrifugation à 34 000 RPM dure seize heures à 4°C dans un rotor SW 41.

MILIEU NaCl (Peeters et al. 1980)

### a) Traitement de l'échantillon

200 µg d'ARN poly A en solution sont chauffés à 65°C pendant cinq minutes puis refroidis brutalement dans la glace fondante.

### b) Centrifugation

L'échantillon traité est déposé au sommet du gradient linéaire 15-35 % saccharose milieu 100 mM NaCl-10 mM TRIS HCl pH 7,6 - 1 mM EDTA. La centrifugation à 38 000 RPM dure dix huit heures à 4°C dans un rotor SW 41.

Après la collecte chaque fraction est précipitée, après un lavage les culots obtenus sont dissous dans un peu d'eau distillée stérile (30 µl), ces solutions sont testées par traduction in vitro.

### 4. Estimation de la taille du messager par fractionnement sur gradient de saccharose

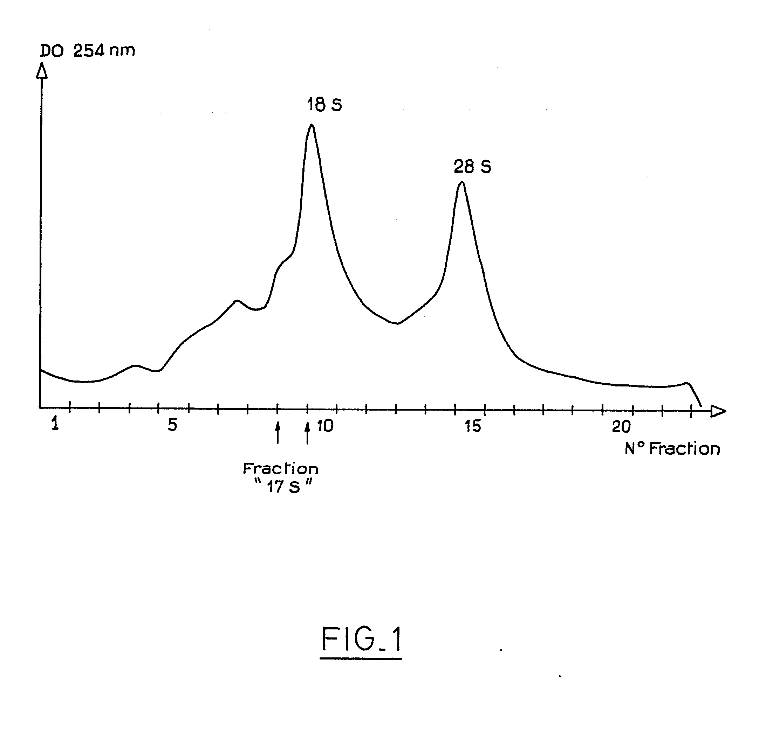

La figure 1 montre le profil des gradients après centrifugation. A l'évidence la colonne d'oligodT n'a pas éliminé tous les ARN ribosomiques encore très représentés par les pics 18 S et 28 S qui nous serviront d'étalons de coefficient de sédimentation. Les résultats des synthèses acellulaires correspondant aux ARN séparés sur gradient montrent que les messagers sont assez bien classés selon la taille du produit pour lequel ils codent. L'ARNm codant pour la protéine 32 000 se situe dans la fraction correspondant à un coefficient de sédimentation de 17 S.

## II. Préparation des sondes d'ADNc

La stratégie utilisée comporte les étapes suivantes :

- Fabrication d'ADNc de la fraction 17 S des ARN polyA de TAB de rat adapté au froid.

- Insertion des séquences d'ADNc dans un plasmide bactérien et obtention de bactéries contenant le plasmide chimère (clones recombinants).

- Sélection de clones bactériens donnant un signal différentiel lorsqu'ils sont mis en contact avec une sonde ADN radioactive préparée soit à partir de polyA de TAB de rat témoin, soit à partir de polyA de TAB thermogénique.

- Sélection d'une séquence ADNc s'hybridant à l'ARNm de la protéine découplante.

Les techniques utilisées sont rappelées ci-après dans le schéma 1.

11

ARN messager

0148686

5'P ～～～～～～～～～～～～AAAAAAAAAAA3'OH
TTTT
oligodT

Synthèse du premier brin      Transcriptase réverse

5'P ～～～～～～～～～～～AAAAAAAAAAA3'OH
3'OH ————————————————TTTT5'P

Hydrolyse de l'ARN

3'OH———————————————— TTTT 5'P

Formation d'une boucle et
autoappariement par l'extrémité 3'OH

TTTT 5'P
3'OH

Synthèse du deuxième brin       Transcriptase réverse

TTTT 5'P
3'OH

Hydrolyse de la boucle       Nucléase $S_1$

3'OH ————————————— 5'P
5'P ————————————— 3'OH

ADNc bicaténaire

Schéma 1

1. <u>Obtention des ADNc double brin</u> (voir schéma 1)

a) <u>Polymérisation d'un premier brin complémentaire de l'ARNm</u>

L'ARNm est formé d'une seule chaine de nucléotides dont une partie de la séquence code pour la protéine. La transcriptase reverse, en face du brin d'ARNm synthétise un premier brin d'ADNc si elle dispose 1) d'une amorce, ici l'oligodT, appariée à la queue polyA de l'ARNm, 2) des désoxynucléotides triphosphate qu'elle va polymériser en libérant les deux phosphates terminaux, 3) d'un milieu ionique adéquat. On marque ce premier brin avec un nucléotide traceur radioactif par son phosphate (d XTP $\alpha^{32}$p) ; il est ainsi facile de suivre cette molécule avec un compteur Geiger et de la quantifier avec un compteur à scintillation. A partir de 3 µg d'ARN polyA de la fraction 17 S du gradient, on synthétise à peu près 1 µg d'ADNc simple brin.

b) <u>Destruction de l'ARNm et récupération du premier brin</u>

L'hydrolyse de l'ARN et de lui seul est possible en milieu alcalin (soude 0,3 N) ; ici elle a duré une nuit à 37°C. Après neutralisation du milieu on sépare les brins d'ADNc des nucléotides non incorporés par filtration sur une colonne de Séphadex G 75. Le premier pic recueilli est précipité en présence de 10 à 40 µg de Dextran dans un tube bien siliconé car l'ADN simple brin a tendance à s'accrocher aux parois. Centrifugé, lavé puis remis en solution ce premier brin est prêt à servir de matrice pour la synthèse du deuxième brin complémentaire, un auto-appariement de son extrémité 3' fournissant à l'enzyme, responsable de cette deuxième réaction, l'amorce nécessaire.

c) Synthèse du deuxième brin

Le deuxième brin a, lui aussi, été polymérisé par la reverse transcriptase. Cette réaction a lieu dans les mêmes conditions que la synthèse du premier brin mais il est inutile d'ajouter l'oligodT.

d) Hydrolyse des segments non appariés

La nucléase S1 attaque spécifiquement les zones non appariées d'un acide nucléique mais respecte les fragments bifilaires. Suite à l'action de cette enzyme, on dispose d'une population d'ADNc formés de molécules bicatenaires dont les deux brins sont appariés sur toute leur longueur.

2. Classement des ADNc bicatenaires par ordre de taille

Il a lieu par centrifugation sur un gradient de saccharose des ADNc bicatenaires obtenus (Rotor SW 50 ; 49 000 RPM ; 5 H). La fraction 9, composée des petits ADNc, supposée plus représentative de la population de départ, est sélectionnée pour réaliser le plasmide chimère. Les autres ADNc (fractions 6, 7 et 8) sont conservés après leur allongement.

3. Réalisation du plasmide chimère (voir schéma 2)

Le plasmide utilisé, pBR 327 (Soberon et al. 1980), est une molécule qui confère aux bactéries qui le portent la résistance à deux antibiotiques, la tétracycline et l'ampicilline ; dans la séquence du gène de résistance à l'ampicilline se trouve le seul site de coupure par l'endonucléase de restriction Pst1 qui transforme ainsi le plasmide circulaire en une molécule linéaire. Pour lier

ADNc double brin

5'P ————————— 3'OH
3'OH ————————— 5'P

dCTP → Terminale transférase

5'P ————— CCCC — CC 3'OH
3'OHCC—CCC ————— 5'P

Plasmide pBR 322

Site de coupure
par Pst 1

3' 5' 3' 5'

dGTP → Terminale transférase

o o·····o o 3'OH
3'OHoo·····ooo
5'P                    5'P

Hybridation

Futur site Pst 1 →

← Futur site Pst 1

Transformation

Clones

ce pBR, linéarisé par Pstl, à l'ADNc double brin, nous allons allonger les extrémités 3' des brins de chacune de ces molécules. pBR sera allongé par des dG, l'ADNc par des dC. Nous ajoutons ainsi des bouts collants qui permettent l'arrimage des deux molécules en un plasmide chimère qui va être utilisé pour transformer les bactéries.

Après cette transformation, la bactérie termine le travail et soude par des liaisons covalentes l'ADNc et le pBR 327, le gène de résistance à l'ampicilline est inactivé par cette insertion. Devenues tétracycline résistantes, ces bactéries restent ampicilline sensibles. Par ailleurs elles ont restauré deux sites de coupure Pstl ce qui permettra ainsi de récupérer l'ADNc flanqué des paires C≡C (appelé aussi insert) par action de cette enzyme.

### 4. Transformation des bactéries

La souche d'Escherichia coli utilisée est la souche 1024 : C200 Rec B, C. Les bactéries cultivées jusqu'à 0,6 unité de D.O. sont traitées au $CaCl_2$ à 0°C, puis mises en contact avec les plasmides chimères préparés précédemment. On les soumet à un choc thermique ; après une demi heure à 37°C, elles sont étalées sur des boîtes de Petri garnies d'un milieu gélosé contenant de la tétracycline ; ce milieu sera donc sélectif. Parallèlement on mène la même transformation avec un aliquot de la préparation de plasmide linéarisé à Pstl et allongé, afin de tester le bruit de fond de cette préparation. Le lendemain les clones bactériens sont visibles sur la gélose : on compare les boîtes de clones porteurs de chimères qui doivent être nombreux, aux boîtes de clones transformés avec la préparation de plasmide linéarisé et allongé qui doivent être beaucoup plus rares. Sur les quelques milliers de clones obtenus avec la préparation de plasmide chimère, une centaine était attribuable à des plasmides non coupés par Pst1.

## 5. Repiquage des bactéries

Les colonies éparpillées au hasard sur les boîtes de Petri sont repiquées en bon ordre sur une boîte de Petri contenant le même milieu gélosé additionné de tétracycline et sur des filtres de nitrocellulose quadrillés 5 x 5 mm reposant sur le même milieu (les bactéries puisent alors leurs nutriments à travers le filtre). Il faut veiller à respecter une exacte correspondance entre la position d'un clone sur la boîte de gélose et sur les filtres. On a ainsi repiqué entre 1 500 et 2 000 clones différents, chaque colonie ayant une réplique sur deux filtres de nitrocellulose distincts. Chaque clone est ainsi "adressé" et il lui correspond un code chiffré. On laisse croître les colonies entre 10 et 24 heures à 37°C.

Par lyse des colonies ayant poussé sur les filtres, suivie d'un traitement approprié, on fixe très solidement l'ADN de ces bactéries, donc les plasmides porteurs de l'ADNc, à la nitrocellulose du filtre. Chacun de ces dépôts d'ADN correspond à un clone, donc à un ADNc fixé à la même adresse que celle de la colonie de bactéries gardées encore vivantes à 4°C dans la boîte de gélose ; celle-ci constitue une bibliothèque de clones longtemps disponibles. Les filtres contenant l'ADN fixé serviront aux expériences d'hybridation.

## 6. Préparations de plasmides

Pour obtenir des plasmides, il faut commencer par cultiver les bactéries dans un milieu liquide contenant de la tétracycline. Deux catégories de méthodes sont ensuite utilisables :

a) les préparations rapides : à partir de quelques millilitres de suspension bactérienne, on peut en moins d'une journée obtenir une solution de plasmide souvent satisfaisante.

b) les techniques lourdes : elles débutent par la préparation d'ADN bactérien et se terminent par un tri du plasmide (superenroulé) sur un gradient de chlorure de cesium qui nécessite une ultracentrifugation de 40 heures.

## 7. Excision et séparation de l'insert ADNc

L'action de l'endonucléase de restriction Pst1 libère l'insert situé entre les deux sites de coupure de cette enzyme, sites reconstitués par la bactérie receveuse. C'est souvent lors de cette coupure enzymatique que les plasmides plus ou moins purs, obtenus par préparation rapide, posent quelques problèmes.

Les inserts sont séparés du vecteur (linéarisé) par électrophorèse en gel d'agarose ou d'acrylamide. Ils sont récupérés par découpage de la fraction de gel les contenant et par électroélution suivie de leur précipitation et dissolution.

## 8. Marquage des inserts

Le marquage des inserts peut être fait soit par des nucléotides radioactifs ou marqués par des éléments fluorescents ou encore par des nucléotides modifiés chimiquement comme décrit dans le brevet EP 63879 ou le FR-A-8124131. Le marquage radioactif des inserts se fait par "Nick Translation". Ce procédé enzymatique utilise les propriétés de l'ADN polymérase 1 d'Escherichia coli qui substitue les désoxynucléotides naturels du plasmide par des désoxynucléotides fournis sous forme triphosphate et dont certains sont radioactifs. Grâce à cette réaction, des sondes très radioactives sont obtenues.

La banque de 1 500 à 2000 clones recombinants "adressés" est conservée à 4°C. Les filtres porteurs des ADNc fixés sont disponibles pour la première opération de sélection (voir 5).

## 9. Sélection d'une banque restreinte de clones différentiels

Le premier mode de sélection consiste à utiliser le fait que l'ARN messager de la protéine découplante est cinq fois plus abondant dans la fraction 17 S du gradient saccharose d'ARN polyA de TAB de rat adapté au froid que dans la fraction homologue obtenue à partir des rats témoins. Par action de la reverse transcriptase sur un aliquot de ces deux fractions, une sonde ADNc très radioactive a été préparée. Un exemplaire de chaque filtre est mis en contact avec la sonde "17 S Froid", son frère jumeau avec la sonde "17 S Chaud" (c'est-à-dire obtenu à partir de rat n'ayant pas été maintenu au froid). Cette incubation permet aux séquences homologues présentes dans la sonde, et sur les filtres de nitrocellulose, de s'apparier spécifiquement. L'ADNc du filtre fixe d'autant plus de sonde radioactive que celle-ci est abondante dans la préparation. Après lavages, séchage et autoradiographie, on étudie la répartition des spots radioactifs sur les filtres. Certains clones se marquent différemment selon que la sonde utilisée est "17 S Froid" ou "17 S Témoin". Un clone, tel celui numéroté 4.5.5, se comporte comme si l'ADNc qu'il contient était complémentaire d'une séquence plus abondante dans "17 S Froid" que dans "17 S Témoin". Ceci correspond donc à un ARNm mieux représenté chez le rat au froid que chez le rat témoin, ce qui est précisément le cas du messager de la protéine découplante. Ce critère a permis de sélectionner 74 clones. Ces clones sont dits "présemptifs", certains d'entre eux peuvent contenir la séquence d'ADNc complémen--taire de celle du messager de la protéine découplante. Ces candidats sont immédiatement repiqués sur une boîte de Petri et sur filtres de nitrocellulose. Ces clones constituent alors une banque restreinte.

### 10. Etude de la banque restreinte

La démarche utilisée pour affiner la sélection parmi les 74 clones différentiels de la banque restreinte repose sur le raisonnement suivant :

Si (hypothèse n° 1), tous ces clones portent des séquences ADNc complémentaires d'une partie de celle du messager de la protéine découplante, un de ces ADNc pris au hasard sera capable de s'hybrider avec tous les autres ou du moins avec une grande partie des ADNc de cette banque restreinte.

Inversement (hypothèse n° 2), si la procédure de sélection différentielle est totalement inefficace nous avons en fait pris 74 clones au hasard pour former notre banque restreinte et, statistiquement, un des inserts pris dans cette banque a peu de chances d'y trouver une autre séquence ADNc avec laquelle il pourrait s'hybrider.

La réalité se situant sans doute entre ces deux solutions extrêmes, nous nous attendions à obtenir un petit nombre de familles de clones.

L'expérience a été faite en utilisant les inserts venant de deux clones (4.5.5. et 3.8.3). On obtient un premier ensemble avec 4.5.5. Puis on compare les ensembles de clones avec lesquels s'hybrident les inserts 4.5.5. et 3.8.3. Ces deux ensembles sont largement chevauchants, leur réunion forme un ensemble de clones apparentés au sein de la banque restreinte : 16 clones sur 74. Le lien de parenté qui les réunit pourrait être une séquence d'ADNc complémentaire de l'ARNm de la protéine découplante.

Les plasmides chimères linéarisés et fixés à de la nitrocellulose sont utilisés pour purifier, par hybridation-déhybridation, l'ARN qui leur a servi de modèle. La traduction de ce dernier indique pour quelle protéine ils codent. Le protocole utilisé est dérivé de celui de Ricciardi et al. (1979).

Les résultats de ces expériences d'hybridation positive confirment la prédiction faite à la fin du paragraphe précédent. Les clones de la famille 3.8.3 4.5.5. sont des clones bactériens ayant intégré une séquence ADNc complémentaire de l'ARNm de la protéine découplante.

Ces clones 3.8.3 et 4.5.5. peuvent être utilisés pour la détection et le dosage des ARNm de la protéine découplante.

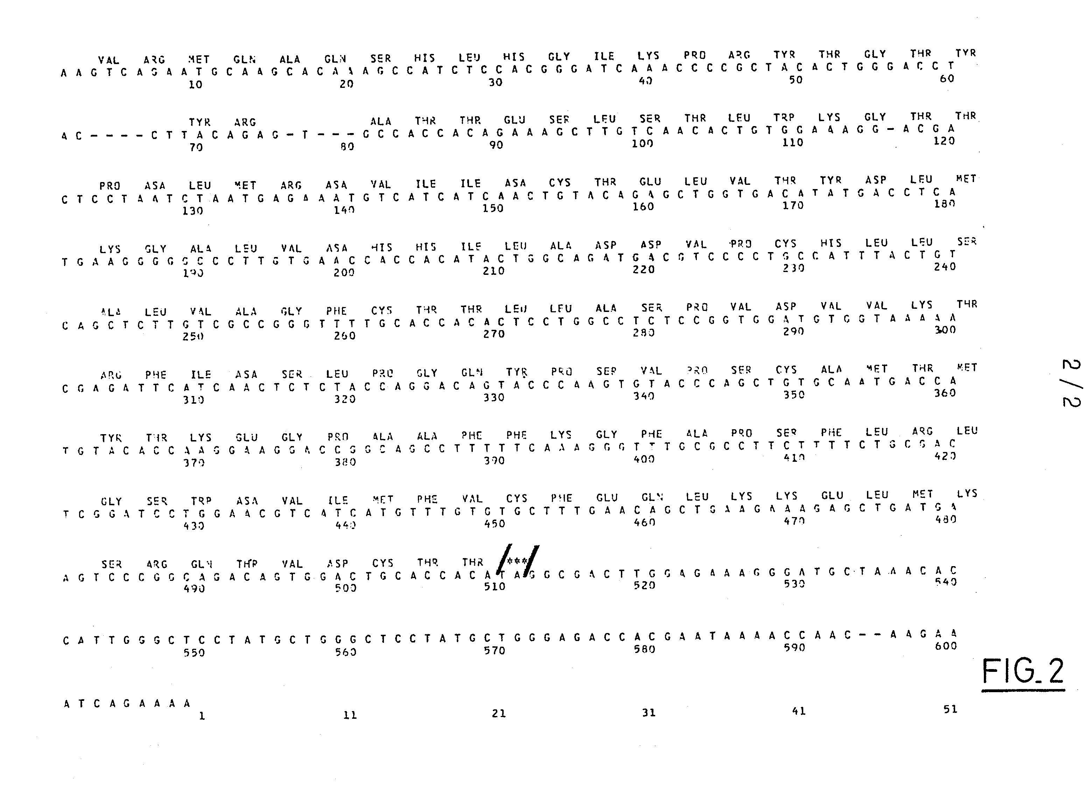

11. La figure 2 représente la séquence C terminale telle qu'elle a pu être déterminée actuellement du cADN de la protéine découplante des mitochondries du tissu adipeux brun de rat.

Cette séquence correspond à environ 160 acides aminés et a un poids moléculaire d'environ 18 000 (soit un peu plus de la moitié de la protéine).

## Bibliographie

AVIV, H. and LEDER, P. (1972) - Purification of biologically active globin messenger RNA by chromatography on oligothymidylic acid-cellulose. Proc. Natl. Acad. Sci. USA 69, 1408-1412.

CANNON, B., HEDIN, A. and NEDERGAARD, J. (1982) - Exclusive occurence of thermogenic antigen in brown adipose tissue, FEBS Lett. 150, 129-132.

HEATON, G.M., WAGENVOORD, R.J., KEMP, A. and NICHOLLS, D.C. (1978) - Brown adipose tissue mitochondria : photoaffinity labelling of the regulatory site of energy dissipation. Eur. J. Biochem. 82, 515-521.

LIN, C.S. and KLINGENBERG, M. (1980) - Isolation of the uncoupling protein from brown adipose tissue mitochondria. FEBS Lett. 113, 299-303.

LOMEDICO, P.T. and SAUNDERS, G.F. (1976) - Preparation of pancreatic mRNA : cell-free translation of an insulin-immunoreactive polypeptide, Nucl. Acids Res. 3, 381-391.

MORY, C., RICQUIER, D., NECHAD, M. and HEMON, P. (1982) - Impairment of trophic response of brown fat to cold in guanethidine-treated rats. Am. J. Physicl. 242, C159-C165.

PEETERS, B.L., MOUS, J.M., ROMBAUTS, W.A. and HEYNS, W.J., (1980) - Androgen-induced messenger RNA in rat ventral prostate. Translation, partial purification and preliminary characterization of the mRNAs encoding the components of prostatic binding protein, J. Biol. Chem. 255, 7017-7023.

RICCIARDI, R.P., MILLER, J. and ROBERTS, B.E. (1979) - Purification and mapping of specific mRNAs by hybridization-selection and cell-free translation, Pro. Natl. Acad. Sci. USA 76, 4927-4931.

RICQUIER, D. and KADER, J.C. (1976) - Mitochendrial protein alteration in active brown fat : a sodium dodecyl sulfate-polyacrylamide gel electrophoretic study. Biochem. Biophys. Res. Commun. 73, 577-583.

RICQUIER, D., MORY, C. and HEMON, P. (1979) - Changes induced by cold adaptation in the brown adipose tissue from several species of rodents, with special reference to the mitochondrial components. Can. J. Biochem. 57, 1262-1266.

RICQUIER, D., NECHAD, M. and MORY, G. (1982 b) - Ultra-structural and biochemical characterization of human brown adipose tissue in pheochromocytema J. Clin. Endocrinol. Metab. 54, 803-807.

RICQUIER, D., LIN, C.S. and KLINGENBERG, M. (1982 a) - Isolation of the GDP binding protein from brown adipose tissue mitochondria of several animals and amino acid composition study in the rat. Biochem. Biophys. Res. Commun. 106, 582-589.

RICQUIER, D., BARLET, J.P., GAREL, J.M., COMBES-GEORGE, M. and DUBOIS, M.P. (1983 a) - An immunological study of the uncoupling protein of brown adipose tissue mitochondria, Biochem. J. 210, 859-866.

RICQUIER, D., MORY, G., NECHAD, M., COMBES-GEORGE, M. and THIBAULT, J. (1983 b) - Development and activation of brown fat in rats with pheochromocytoma PC 12 tumors. Am. J. Physiol. 245, C172-C177.

SCHWEINFEST, C.W., KWIATKOWSKI, R.W. and DOTTIN, R.P. (1982) - Molecular cloning of a DNA sequence complementary to creatine kinase M mRNA from chickens, Proc. Natl. Acad. Sci. USA 79, 4997-5000.

SOBERON, X., COVARRUBIAS, L. and BOLIVAR, F. (1980) - Construction and characterization of new cloning vehicles. V Deletion derivatives of pBR 322 and pBR 325, Gene 9, 287-305.

0148686

## REVENDICATIONS

1. Sonde constituée en partie d'une séquence d'acides nucléiques, cette séquence d'acide nucléique reconnaissant spécifiquement l'ARNm et/ou le gène de la protéine découplante des mitochondries du tissu adipeux brun des mammifères.

2. Sonde selon la revendication 1 caractérisée en ce que la séquence d'acides nucléiques est une séquence d'ADNc.

3. Sonde selon l'une des revendications 1 et 2 caractérisée en ce qu'elle comporte en outre tout ou partie de l'ADN d'un plasmide bactérien.

4. Sonde selon la revendication 3 caractérisée en ce que le plasmide bactérien est le plasmide pBR 327.

5. Sonde selon l'une des revendications 1 à 4 caractérisée en ce qu'elle comporte un élément de marquage.

6. Sonde selon la revendication 5 caractérisée en ce que l'élément de marquage est un marqueur radioactif.

7. Procédé d'identification et/ou de dosage des ARNm de la protéine découplante des mitochondries du tissu adipeux brun des mammifères caractérisé en ce qu'on met en contact un milieu comportant lesdits ARNm avec une sonde selon l'une des revendications 1 à 6 et que l'on identifie et/ou dose la sonde fixée.

8. Procédé de préparation d'une sonde d'ADNc selon la revendication 2 caractérisé en ce que :
   - on prélève la fraction 17 S des ARN polyA de TAB d'animaux adaptés au froid,
   - on fabrique des copies d'ADNc desdits ARN,
   - on insert les séquences d'ADNc dans un plasmide,
   - on transforme les bactéries à l'aide du plasmide obtenu,
   - on sélectionne les clones bactériens portant la séquence d'ADNc s'hybridant à l'ARNm de la protéine découplante.

9. Ensemble d'identification et/ou de dosage pour la mise en oeuvre du procédé selon la revendication 7 qui comporte au moins une sonde selon l'une des revendications 1 à 6.

FIG_1

```
      VAL   ARG   MET   GLN   ALA   GLN   SER   HIS   LEU   HIS   GLY   ILE   LYS   PRO   ARG   TYR   THR   GLY   THR   TYR
    A A G T C A G A A T G C A A G C A C A A A G C C A T C T C C A C G G G A T C A A A A C C C C G C T A C A C T G G G A C C T
            10                  20                  30                  40                  50                  60

                  TYR   ARG               ALA   THR   THR   GLU   SER   LEU   SER   THR   LEU   TRP   LYS   GLY   THR   THR
    A C - - - - C T T A C A G A G - T - - - G C C A C C A C A G A A A G C T T G T C A A C A C T G T G G A A A A G G - A C G A
            70                  80                  90                  100                 110                 120

      PRO   ASA   LEU   MET   ARG   ASA   VAL   ILE   ILE   ASA   CYS   THR   GLU   LEU   VAL   THR   TYR   ASP   LEU   MET
    C T C C T A A T C T A A T G A G A A A T G T C A T C A T C A A C T G T A C A G A G C T G G T G A C A T A T G A C C T C A
            130                 140                 150                 160                 170                 180

      LYS   GLY   ALA   LEU   VAL   ASA   HIS   HIS   ILE   LEU   ALA   ASP   ASP   VAL   PRO   CYS   HIS   LEU   LEU   SER
    T G A A G G G G C C C C T T G T G A A C C A C C A C A T A C T G G C A G A T G A C G T C C C C T G C C A T T T A C T G T
            190                 200                 210                 220                 230                 240

      ALA   LEU   VAL   ALA   GLY   PHE   CYS   THR   THR   LEU   LEU   ALA   SER   PRO   VAL   ASP   VAL   VAL   LYS   THR
    C A G C T C T T G T C G C C G G G T T T T G C A C C A C A C T C C T G G C C T C T C C G G T G G A T G T G G T A A A A A
            250                 260                 270                 280                 290                 300

      ARG   PHE   ILE   ASA   SER   LEU   PRO   GLY   GLN   TYR   PRO   SER   VAL   PRO   SER   CYS   ALA   MET   THR   MET
    C G A G A T T C A T C A A C T C T C T A C C A G G A C A G T A C C C A A G T G T A C C C A G C T G T G C A A T G A C C A
            310                 320                 330                 340                 350                 360

      TYR   THR   LYS   GLU   GLY   PRO   ALA   ALA   PHE   PHE   LYS   GLY   PHE   ALA   PRO   SER   PHE   LEU   ARG   LEU
    T G T A C A C C A A G G A A G G A C C C G G C A G C C T T T T T C A A A G G G T T T G C G C C T T C T T T T C T G C G A C
            370                 380                 390                 400                 410                 420

      GLY   SER   TRP   ASA   VAL   ILE   MET   PHE   VAL   CYS   PHE   GLU   GLN   LEU   LYS   LYS   GLU   LEU   MET   LYS
    T C G G A T C C T G G A A C G T C A T C A T G T T T G T G T G C T T T G A A C A G C T G A A G A A A G A G C T G A T G A
            430                 440                 450                 460                 470                 480

      SER   ARG   GLN   THR   VAL   ASP   CYS   THR   THR   /***/
    A G T C C C G G C A G A C A G T G G A C T G C A C C A C A T A G G C G A C T T G G A G A A A G G G A T G C T A A A C A C
            490                 500                 510                 520                 530                 540

    C A T T G G G C T C C T A T G C T G G G C T C C T A T G C T G G G A G A C C A C G A A T A A A A C C A A C - - A A G A A
            550                 560                 570                 580                 590                 600

    A T C A G A A A A
             1                  11                  21                  31                  41                  51
```

FIG.2

0148686